# EUROPÄISCHE PATENTSCHRIFT

(11) **EP 2 449 528 B1**
(45) Veröffentlichungstag und Bekanntmachung des Hinweises auf die Patenterteilung: **22.11.2017**
(21) Anmeldenummer: 10724535.9
(22) Anmeldetag: 22.06.2010
(51) Int. Cl.: A61B 6/03, G01R 33/48, G01R 33/56, G06T 7/11, G06T 11/00

(54) **WISSENSBASIERTE SEGMENTIERUNG SCHWÄCHUNGSRELEVANTER REGIONEN DES KOPFES**
KNOWLEDGE-BASED SEGMENTATION OF WEAKENING-RELEVANT REGIONS OF THE HEAD
SEGMENTATION BASÉE SUR LA CONNAISSANCE DE ZONES D'AFFAIBLISSEMENT AU NIVEAU DE LA TÊTE

(30) Priorität: 03.07.2009 DE 102009027448
(43) Veröffentlichungstag der Anmeldung: 09.05.2012
(73) Patentinhaber: Forschungszentrum Jülich GmbH, 52425 Jülich (DE)
(72) Erfinder: WAGENKNECHT, Gudrun, 52222 Stolberg (DE)
(74) Vertreter: Gille Hrabal
(86) Internationale Anmeldenummer: PCT/EP2010/058811
(87) Internationale Veröffentlichungsnummer: WO 2011/000739

(56) Entgegenhaltungen:
- WAGENKNECHT G ET AL: "Wissensbasierte Segmentierung schwaechungsrelevanter Regionen des Kopfes in T1-gewichteten MRT-Bilddaten fuer die Schwaechungskorrektur in MR-PET Systemen", NUKLEARMEDIZIN, SCHATTAUER VERLAG, STUTTGART, DE, Bd. 48, Nr. 2, 22. April 2009 (2009-04-22) , Seite V147, XP009136171, ISSN: 0029-5566
- WAGENKNECHT G ET AL: "MRI-based individual 3D region-of-interest atlases of the human brain: a new method for analyzing functional data.", METHODS OF INFORMATION IN MEDICINE 2004 LNKD- PUBMED:15472751, Bd. 43, Nr. 4, 2004, Seiten 383-390, XP002592300, ISSN: 0026-1270
- WAGENKNECHT GUDRUN ET AL: "Individual 3D region-of-interest atlas of the human brain: Knowledge-based class image analysis for extraction of anatomical objects", 2000, PROCEEDINGS OF SPIE - THE INTERNATIONAL SOCIETY FOR OPTICAL ENGINEERING 2000 SOCIETY OF PHOTO-OPTICAL INSTRUMENTATION ENGINEERS, VOL. 3979, PAGE(S) I/, XP002592301, das ganze Dokument
- GUDRUN WAGENKNECHT ET AL: "Knowledge-based segmentation of attenuation-relevant regions of the head in T1-weighted MR images for attenuation correction in MR/PET systems", 2009 IEEE NUCLEAR SCIENCE SYMPOSIUM AND MEDICAL IMAGING CONFERENCE (NSS/MIC 2009), IEEE, ORLANDO, FL, USA, 24. Oktober 2009 (2009-10-24), Seiten 3338-3343, XP031621139, ISBN: 978-1-4244-3961-4
- HELMS G ET AL: "Contrast-driven approach to intracranial segmentation using a combination of T2- and T1-weighted 3D MRI data sets", JOURNAL OF MAGNETIC RESONANCE IMAGING WILEY UK, Bd. 24, Nr. 4, Oktober 2006 (2006-10), Seiten 790-795, XP002610164, ISSN: 1053-1807
- ZEIN SALAH ET AL: "Preoperative planning of a complete mastoidectomy: semiautomatic segmentation and evaluation", INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE, Bd. 1, Nr. 4, 23. November 2006 (2006-11-23), Seiten 213-222, XP019456771, ISSN: 1861-6429, DOI: DOI:10.1007/S11548-006-0057-1

## Beschreibung

Die vorliegende Erfindung betrifft ein Verfahren für die Ermittlung von Schwächungsbereichen in einem Körper,

Die Positronen-Emissions-Tomographie (Abkürzung PET) ist ein bildgebendes Verfahren der Nuklearmedizin, das Schnittbilder von lebenden Organismen erzeugt, indem es die Verteilung einer schwach radioaktiv markierten Substanz im Organismus sichtbar macht und damit biochemische und physiologische Funktionen abbildet,

Zu Beginn einer Untersuchung wird dem zu untersuchenden Lebewesen ein Radiopharmakon verabreicht und zwar Radionuklide, die Positronen emittieren (Betastrahlung), Bei der Wechselwirkung eines Positrons mit einem Elektron im Körper werden zwei hochenergetische Photonen in genau entgegengesetzte Richtungen, also mit dem Winkel 180 Grad zueinander, ausgesandt (Vernichtungsstrahlung), Die PET-Vorrichtung enthält ringförmig um den Patienten angeordnete Detektoren für die Photonen. Registrieren zwei genau gegenüberliegende Detektoren zeitgleich ein Photon, so wird dieses Ereignis einem Zerfall zugeordnet, der auf einer geraden Linie zwischen den beiden Detektoren stattgefunden hat, Aus der sich so ergebenden zeitlichen und räumlichen Verteilung von registrierten Zerfallsereignissen wird auf die räumliche Verteilung des Radiopharmakons im Körperinneren geschlossen und eine Serie von Schnittbildern errechnet, Häufige Anwendung findet die PET bei stoffwechseibezogenen Fragestellungen in der Onkologie, Neurologie sowie Kordiologie,

Die Magnetresonanztomographie (Abkürzung MRT) ist ein bildgebendes Verfahren, das in der medizinischen Diagnostik zur Darstellung von Struktur und Funktion der Gewebe und Organe im menschlichen oder tierischen Körper eingesetzt wird. Die Magnetresonanztomographie basiert auf sehr starken Magnetfeldern sowie elektromagnetischen Wechselfeldern im Radiofrequenzbereich, mit denen bestimmte Atomkerne (meistens die Wasserstoffkerne/Protonen) im Körper angeregt werden. Empfangen werden extrem schwache elektromagnetische Felder, die von den angeregten Atomkernen ausgesendet werden. Eine wesentliche Grundlage für den Bildkontrast sind unterschiedliche Relaxationszeiten verschiedener Gewebearten, Daneben trägt auch der unterschiedliche Gehalt an Wasserstoff-Atomen in verschiedenen Geweben (z. B. Muskel, Knochen) zum Bildkontrast bei.

Aus den Druckschriften "MRI-Based Individual 3D Region-of-Interest Atlases of the Human Brain", G. Wagenknecht, H.-J. Kaiser, U. Buell, O, Sabri, Methods Inf Med 4/2004, Seiten 383 - 390; individual 3D region-of-interest atlas of the human brain: automatic training point extraction for neural network based classification of brain tissue types",G. Wagenknecht, H.-J. Kaiser , T. Obladen, O, Sabri, U. Buell, SPIE Electronic Imaging 2000, San Jose, Proc SPIE 3962: 150-161 ; "individual 3D region-of-interest atlas of the human brain: neural network-based tissue classification with automatic training point extraction", G. Wagenknecht, H.-J. Kaiser, T. Obladen, O, Sabri, U. Buell, SPIE Medical Imaging 2000, San Diego, Proc SPIE 3979: 306-317, ist bekannt, die durch eine MR-Messung erhaltenen Bildinformationen in Gewebeklassen wie grauer Substanz, weißer Substanz, Liquor (Hirnflüssigkeit), Fettgewebe und Hintergrund einzuordnen, um so ein anatomisches Bild vom untersuchten Organismus zu erhalten.

Eine Positronen-Emissions-Tomographie ermöglicht lediglich eine Messung von Funktionen, zum Beispiel in Bezug auf Durchblutung oder Stoffwechsel. Eine Strukturinformation kann mit Hilfe der Positronen-Emissions-Tomographie nicht erhalten werden. Um ergänzend Strukturinformationen zu erhalten, wird zusätzlich eine MR-Messung und damit eine morphologische Messung durchgeführt. Es wird so u. a, ermittelt, wo welche Gewebebereiche beispielsweise im Fall von einem Gehirn aufzufinden sind, so dass eine Zuordnung zwischen Funktion und Anatomie möglich wird. Mit Hilfe einer MR-Messung können also beispielsweise die Morphologie eines Kniegelenks oder von Organen sichtbar gemacht werden, was mit der Positronen-Emissions-Tomographie nicht möglich ist.

In der Regel werden heutzutage MRT- und PET-Messungen in zwei verschiedenen Vorrichtungen durchgeführt, nämlich zum einen in einem Kernspintomographen zur Durchführung einer Magnetresonanztomographie und zum anderen in einem Positronen-Emissions-Tomographen, Es gibt aber auch Vorrichtungen - nachfolgend MR/PET-Vorrichtungen genannt - (beispielsweise bekannt aus der WO 2008/006451 A), die sowohl einen Kernspintomographen als auch einen Positronen-Emissions-Tomographen umfassen, um so beide eingangs genannten Messungen durchführen zu können, ohne dafür den zu untersuchenden Organismus bewegen zu müssen. Werden nämlich die beiden Messungen in unterschiedlichen Vorrichtungen durchgeführt, so gelingt es praktisch nie, einen zu untersuchenden Organismus in gleicher Weise so zu positionieren, dass die von beiden Vorrichtungen erhaltenen Bilddaten sich auf dieselbe räumliche Orientierung beziehen, Es muss daher dann versucht werden, die Bilddatensätze einer MR-Messung an die Bilddatensätze einer PET-Messung hinsichtlich der räumlichen Orientierung aneinander anzupassen (zu registrieren), um biochemische und / oder physiologische Funktionen anatomisch zuordnen zu können. Mit einer Vorrichtung, die sowohl eine MRTals auch eine PET-Untersuchung ermöglicht, ohne dafür ein Organismus bewegen zu müssen, vermeidet man solche Anpassungserfordernisse,

Eine im Rahmen von PET- oder MRT-Untersuchungen zu detektierende Strahlung tritt im Inneren eines Organismus auf und wird von außerhalb des Organismus angeordneten Detektoren erfasst. Da die Strahlung auf dem Weg vom Entstehungsort zum Detektor Bereiche des Organismus wie Weichteilgewebe, Knochen sowie luftgefüllte Hohlräume passieren muss, wird diese von diesen Bereichen (nachfolgend auch Schwächungsregionen genannt) geschwächt und damit unvollständig von den Detektoren erfasst. Die verschiedenen Schwächungsregionen schwächen eine Strahlung unterschiedlich stark.

Problematisch an einer solchen Schwächung von Strahlung ist, dass hierdurch Ergebnisse verfälscht werden, wenn der Einfluss einer Schwächung nicht korrigiert bzw. nicht berücksichtigt wird. Im Fall einer Positronen-Emissions-Tomographie können beispielsweise aufgrund von Schwächung abweichend von der Wirklichkeit im Rahmen einer Messung nur wenige Zerfälle durch die entsprechenden Detektoren erfasst worden sein. Um in einem solchen Fall die wahre Zahl an Zerfällen ermitteln und damit eine zutreffende Information erhalten zu können, ist es erforderlich, Veränderungen aufgrund von Schwächungen einschätzen und korrigieren zu können, (siehe R. Standke: Technische Grundlagen der 18F-Fluorodeoxyglukose-Positronen-emissionstomographie-Diagnostik; Acta Medica Austriaca, Blackwell Verlag, 29. Jahrgang, Heft 5 2002, S. 149-155; siehe auch http;//de.wikipedia.org/wiki/Positronen-Emissions-Tomographie#Korrektur_der_Messdaten (gemessene Schwächungskorrektur mit Stabquellen); C. Burger, G. Goerres, S. Schoenes, PET attenuation coefficients from CT images: experimental evaluation of the transformation of CT into PET 511 -keV attenuation coefficients. EJNMMI, 29:922-927, 2002. (PET-CT: berechnete Schwächungskorrektur); M. Hofmann, et al, MRI-Based Attenuation Correction for PET/MRI; A Novel Approach Combining Pattern Recognition and Atlas registration. JNM, 49; 1875-1883, 2008. (MR/PET; berechnete Schwächungskorrektur) ; Tyler DJ, Robson MD, Henkelman RM, Young IR, and Bydder GM Magnetic Resonance Imaging with Ultrashort TE (UTE) Pulse Sequences: Technical Considerations J Magn Reson Imaging (2007) 25:279-289 (Ziel ist die Entwicklung neuer Sequenzen, die eine verbesserte Abgrenzung von Knochengewebe erlauben)).

Um eine Schwächung einer detektierten Strahlung korrigieren zu können, ist es erforderlich zu wissen, welche Schwächungsregionen eine Strahlung passieren musste, um zum entsprechenden Detektor zu gelangen. Die dafür erforderlichen Bilddaten, die die dafür benötigten anatomischen Informationen liefern, können wie erwähnt von einer MRT-Messung stammen, Mithilfe dieser Bilddaten können allerdings nicht alle interessierenden Schwächungsregionen bzw. Schwächungsbereiche auf Basis der intensitätswerte (Grauwerte) unterschieden werden, So ist es nicht möglich, Knochenbereiche und luftgefüllte Hohlräume voneinander zu unterscheiden, da sie im gleichen Grauwertbereich abgebildet werden, Dies wäre aber erforderlich, um zutreffend ermitteln zu können, ob eine Strahlung durch einen Knochen geschwächt wurde oder einen luftgefüllten Hohlraum passiert hat,

Aus der Druckschrift "Wagenknecht G. et al., Wissenbasierte Segmentierung schwächungsrelevanter Regionen des Kopfes in T1-gewichteten MRT-Bilddaten für die Schwächungskorrektur in MR-PET Systemen, Nuklearmedizin, Schattauer Verlag, Studttgart, DE, Bd. 48, Nr. 2, 22, April 2009" geht eine Methode mit Eingangsdaten hervor, bei denen es sich um T1 gewichtete MRT-Bilddaten eines zu segmentierenden Kopfes handelt, In dieser Druckschrift wird angegeben, Signal-, Lage- und Formeigenschaften zur Differenzierung von Regionen mit unterschiedlichen Schwächungsbereichen im Rahmen einer wissensbasierten Bilddatenanalyse zu nutzen und zwar unter gleichzeitiger Verwendung eines Klassifzierungsverfahrens, Als Ergebnis können zerebrales und extrazerebrales Weichteilgewebe klassifiziert werden, Zudem offenbart diese Druckschrift, dass mit dieser Methode eine dreidimensionale Detektion und Segmentierung der Nasennebenhöhlen sowie der Mastoidzellregionen möglich sel,

Es ist Aufgabe der vorliegenden Erfindung, Schwächungsbereiche verbessert zu ermitteln, um die MR/PET-Technik weiter entwickeln zu können.

Gelöst wird die Aufgabe durch ein Verfahren nach Anspruch 1,

Zur Lösung der Aufgabe wird ein tierischer oder menschlicher Körper ganz oder teilweise mittels Magnetresonanztomographie erfasst, so dass ein MRT-Bilddatensatz erhalten wird. Die Grauwerte des MRT-Bilddatensatzes werden derart klassifiziert, dass jeder Grauwert einer Gewebeklasse zugeordnet wird. Aus oben genannten Gründen gelingt eine solche erste Zuordnung nur näherungsweise, da nicht alle Schwächungsbereiche aufgrund der Grauwerte eindeutig identifiziert werden können. Daher werden anschließend die auf Basis der Grauwerte klassifizierten Voxel des Bilddatensatzes mit der Anatomie des untersuchten Körpers bzw, des untersuchten Körperteils verglichen, um zu überprüfen, ob das Ergebnis der Klassifizierung plausibel ist. Ergibt der Vergleich, dass das Ergebnis der grauwertbasierten Klassifizierung eines Voxels nicht mit der Anatomie des untersuchten Körpers bzw, des untersuchten Körperteils übereinstimmen kann, so wird das Voxel so umklassifiziert, dass kein Widerspruch mehr zwischen der Einordnung in eine Gewebeklasse und der bekannten Anatomie besteht. Es findet also eine Lfmklassifizierung in Abhängigkeit vom durchgeführten Vergleich statt,

Schwächungsbereiche in einem Körper können so verbessert ermittelt werden. Die so verbesserte Kenntnis über die Lage und Ausdehnung der Schwächungsbereiche im untersuchten Körper bzw. im untersuchten Körperteil erlaubt es, verbessert die Schwächung einer Strahlung ermitteln und korrigieren zu können.

Würde man multispektrale MRT-Bilddaten nutzen, also mehrere Sequenzen, würde man mehrere Parameter (Grauwerte) pro Voxel haben (also einen Vektor), mit dem man klassifizieren würde, Auch in solchen Fällen kann die Erfindung entsprechend angewendet werden.

In einer Ausführungsform der Erfindung wird ein Körper eines Lebewesens mit Hilfe der Positronen-Emissions-Tomographie ganz oder teilweise untersucht und so ein PET-Bilddatensatz erhalten. Außerdem wird das Lebewesen ganz oder teilweise mit Hilfe der Magnetresonanztomographie untersucht und ein MRT-Bilddatensatz erhalten. Wird ein Lebewesen nur teilweise untersucht, so beziehen sich die PET- und die MRT-Untersuchungen auf den gleichen Bereich, so zum Beispiel auf den Kopf des Lebewesens. Bevorzugt finden beide Messungen in einer MR/PET-Vorrichtung statt. Andernfalls sind die beiden Bilddatensätze so aneinander anzupassen (zu registrieren), dass sich die beiden Bilddatensätze auf die gleiche räumliche Orientierung beziehen.

Die PET-Bilddaten dienen der Darstellung der Funktion in einem Organ (wie oben schon erwähnt) und diese PET-Bilddaten werden schwächungskorrigiert, Würde man dies nicht tun, so würde die gemessene Funktion (z.B. Stoffwechsel oder Durchblutung) nicht korrekt ermittelt, was wiederum Auswirkung auf die Diagnostik hat (Z. B. Stoffwechsel durch Schwächung zu gering -> Unterschätzung -> im schlimmsten Fall Fehldiagnose). Mit der vorliegenden Erfindung werden die maßgeblichen Schwächungsregionen aus den MRT-Bilddaten verbessert ermittelt. So gelingt es, PET-Bilddaten verbessert entsprechend zu korrigieren.

Ein so erhaltener MRT-Bilddatensatz umfasst eine Vielzahl von Voxeln, denen jeweils ein Grauwert zugeordnet ist. Die Grauwerte werden nun wie erwähnt klassifiziert, das heißt, jeder betrachtete Grauwert wird als einem bestimmten Bereich zugehörig (nachfolgend "Gewebeklasse" genannt) interpretiert und entsprechend zugeordnet. Wird beispielsweise ein Kopf untersucht, so werden die Grauwerte eines MRT-Bilddatensatzes als graue Substanz, weiße Substanz, Liquor (Hirnflüssigkeit), Fettgewebe und Hintergrund interpretiert und entsprechend zugeordnet. Für die Durchführung der Klassifikation werden vorzugsweise T1 -gewichtete Messergebnisse, die auf Basis von 3D-Sequenzen generiert wurden, verwendet, da diese besonders hochauflösend sind, die wesentlichen Gewebearten kontrastreich darstellen und ein gutes Signal-/Rausch-Verhältnis bei einer für den Patienten akzeptablen Messzeit besitzen, Diese Daten sind damit prädestiniert für die Segmentierung anatomischer Strukturen, Ist eine derart hohe Genauigkeit nicht erforderlich, können auch alternative MR-Sequenzen oder Kombinationen von Bilddaten, die von verschiedenen Sequenzen stammen, eingesetzt werden. Wichtig ist hierbei, dass die zu segmentierenden Bereiche kontrastreich dargestellt werden.

Zur Durchführung der Klassifizierung wird in einer Ausführungsform der Erfindung zunächst ein Bereich bzw. ein Schnitt des untersuchten Lebewesens betrachtet und analysiert, der sämtliche Gewebeklassen umfasst. Im Fall eines Kopfes wird also ein Schnitt durch den Kopf betrachtet, der die Gewebeklassen graue Substanz, weiße Substanz, Liquor (Hirnflüssigkeit), Fettgewebe, Hintergrund bekanntermaßen umfasst. Hierfür geeignet ist beispielsweise ein mittlerer transaxialer Schnitt durch ein menschliches Gehirn, In dieser Schnittebene finden sich die fünf genannten Gewebeklassen, deren Anatomie, und damit auch deren Eigenschaften, wie Homogenität, Größe und räumliche Lage in Relation zueinander bekannt ist. Zunächst beschränkt auf diesen Bereich werden die Grauwerte der Voxel, die zu dem Schnitt gehören, näherungsweise klassifiziert. Es wird also basierend auf der bekannten Anatomie ermittelt, welcher Grauwert welcher Gewebeklasse zuzuordnen ist. Ist das Klassifikationsverfahren auf diese Weise hinreichend trainiert worden, so werden anschließend basierend auf dieser Zuordnung die übrigen Voxel des MRT-Bilddatensatzes auf Basis ihrer Grauwerte klassifiziert.

Als Ergebnis der Gewebeklassifikation wird ein Datensatz 1 eines Klassenbildes erhalten, in dem jedes Voxel einer Gewebeklasse, also im Fall eines Kopfes einer der Klassen graue Substanz (GM), weiße Substanz (WM), Liquor (CSF), Fettgewebe (AT) und Hintergrund (BG) zugeordnet ist.

In einer Ausführungsform der Erfindung werden sämtliche Voxel betrachtet, die als Hintergrund (BG) klassifiziert worden sind und es wird ermittelt, ob ein als Hintergrund klassifiziertes Voxel tatsächlich außerhalb des untersuchten Körpers bzw. Körperbereichs liegt. Ergibt diese Untersuchung, dass ein Voxel innerhalb des untersuchten Körpers liegt, und dennoch aufgrund seines Grauwertes als Hintergrund klassifiziert wurde, so wird dieser geeignet neu klassifiziert und zwar in Abhängigkeit von der Anatomie des untersuchten Körpers bzw. des untersuchten Körperteils. Die Umklassifizierung erfolgt so, dass die Einordnung in eine andere Gewebeklasse sich nicht im Widerspruch zur bekannten Anatomie des untersuchten Körpers bzw. des untersuchten Körperbereichs befindet.

Ist ein Voxel als Hintergrund (BG) klassifiziert worden, so soll es sich dabei um einen außerhalb des Körpers liegenden Bereich handeln. In einer Ausführungsform der Erfindung werden sämtliche Voxel ermittelt, die außerhalb des betrachteten, bekannten Körpers bzw. außerhalb des betrachteten, bekannten Körperbereichs liegen. Falls sich darunter Voxel befinden, die aufgrund ihres Grauwertes nicht als Hintergrund klassifiziert worden sind, so werden diese neu klassifiziert bzw. umgelabelt und so der Klasse BG zugeordnet.

### Schritt 1 ;

In einer Ausführungsform der Erfindung wird im Fall der Untersuchung eines Kopfes in jeder Schicht des Datensatzes zunächst der außerhalb des Kopfes liegende Bereich der Gewebeklasse BG beispielsweise durch ein 2D Region-Growing erfasst. Die noch verbleibenden beispielsweise im Region-Growing nicht erfassten BG-Voxel liegen somit innerhalb des Kopfes und werden nach CSF umgelabelt, da sie nicht Hintergrund sein können. Anschließend wird beispielsweise mit einem 3D Region-Growing der Bereich des Kopfes erfasst. Alle nicht zum Kopf gehörenden Voxel werden anschließend der Gewebeklasse BG zugeordnet. Hierdurch werden Fehlklassifikationen im Bereich des Hintergrundes, die z,B, durch Rauschen oder Bewegungsartefakte bei der Patientenmessung bedingt sein können, beseitigt.

Region-Growing ist ein Bildsegmentierungsverfahren. Bei diesem Verfahren werden homogene Bildelemente zu Regionen verschmolzen, Zuerst wird das Bild in initiale Zellen (1 ×1x1, 2x2x2 oder 4x4x4 Voxel) unterteilt. Beginnend mit der Wahl einer initialen Zelle als Anfangsregion wird diese dann mit den Nachbarzellen verglichen, wobei z.B. die Mittelwerte der Grauwerte der Region und der Nachbarzelle betrachtet werden können. Falls diese ähnlich sind, werden sie verschmolzen, In der vorliegenden Erfindung wird die Klassenzugehörigkeit der Voxel betrachtet, und verschmolzen, wenn die gewünschte Klassenzugehörigkeit vorliegt. Durch den Vergleich mit all seinen Nachbarn wächst die Region weiter. Wenn keine Nachbarn mehr hinzugenommen werden können, ist eine Region gefunden und man wählt eine neue Zelle, welche noch nicht zu einer Region gehört und fängt wieder von vorne an. Der ganze Prozess wird solange wiederholt, bis alle Pixel bzw. Voxel Regionen zugeordnet wurden.

### Schritt 2:

Zur Trennung der extrazerebralen Region von der zerebralen Region werden in einer bevorzugten Ausführungsform als erstes die beiden Augenregionen des Kopfes detektiert und segmentiert, da hier eine ausgeprägte Verbindung aufgrund des Sehnerven zwischen beiden Regionen besteht. Diese Regionen liegen im mittleren Schichtbereich des Kopfes lateral/anterior und sind aufgrund des Corpus adiposum orbitae von Fettgewebe umgeben. Daher erfolgt die Detektion mittels eines angemessen großen und geformten, vorzugsweise quadratischen transaxialen Templates der Gewebeklasse AT. Ein Template ist ein Referenzobjekt (hier einer bestimmten Form und Klasseneigenschaft), das über einen Bereich des Bildes bewegt wird. An jeder Position des Templates werden alle Voxel des Bildes, die vom Template überstrichen werden, mit dem jeweiligen Voxel des Templates verglichen (also hier die Klasseneigenschaft). (Das grundsätzliche Prinzip ist bekannt und geht zum Beispiel aus der Internetseite http://en.wikipedia.org/wiki/Template_matching hervor (Stand 18. Juni 2009). In unserem Fall ist das Template allerdings kein Bildauschnitt mit komplexem Bildinhalt, sondern mit einer speziellen Klasseneigenschaft.) "Angemessen groß" bezieht sich in diesem Zusammenhang auf die ungefähr zu erwartende Größe und (sehr) grobe Form einer anatomischen Region.

Die Bilddaten liegen in einer dreidimensionalen Matrix vor, in der jedes Voxel der Matrix ein Klassenlabel besitzt. Das Template wird im mittleren / vorderen Bereich der dreidimensionalen Bild-Matrix von vorne ausgehend auf der linken und rechten Seite (anatomisch: lateral) solange verschoben, bis auf beiden Seiten jeweils unabhängig voneinander die Region der Klasse AT gefunden ist, bei der die größte Anzahl der Voxel des AT-Templates mit Voxeln der AT-Region überlagert ist. Alle vom Template überlagerten Voxel der Klasse AT auf der rechten und linken Seite werden mit dem Klassenlabel "AUGE" versehen. Der Bereich des Fettgewebes wird anschließend durch 3D Region-Growing komplett erfasst und als AUGE gelabelt. Anschließend wird dieser Bereich in den Bereich WM und dann in den Bereich GM hinein - dilatiert, um so die Augenregion inklusive Sehnerven zu erfassen.

Dilatieren bedeutet, dass der Bereich an den Rändern erweitert wird (s.a. http://de.wikipedia.org/wiki/Dilatation_(Bildverarbeitung) Stand 18. Juni 2009). Hierzu wird ein Strukturelement gewählt und mit diesem verglichen (z.B. ein 3x3x3 großes Element). Dieser Vorgang der Dilatation kann iterativ wiederholt werden.

### Schritt 3:

Im Anschluss daran wird in einer Ausgestaltung des Verfahrens als nächstes die Hirn-Region vom extrazerebralen Bereich durch Erosion getrennt. Erosion ist genau wie die Dilatation eine Basisoperation der morphologischen Bildverarbeitung. Erosion wird mit Hilfe eines Stukturelementes realisiert (z.B. ein 3x3x3 großes Element). Für jedes Strukturelement wird ein Bezugspunkt definiert, welcher das Platzieren des Elementes an einer bestimmten Voxelposition erlaubt. Die eigentliche Operation besteht aus der voxelweisen Verschiebung des Strukturelementes über das Gesamtbild. Es wird geprüft:Passt das strukturierende Element vollständig in die Menge?

Kann die Frage mit ja beantwortet werden, so gehört das Voxel des Bildes, an der Stelle an der sich der Bezugspunkt des Strukturelementes befindet, zur erodierten Menge(vergleiche http://de.wikipedia.org/wiki/Erosion_(Bildverarbeitung) Stand 18. Juni 2009).

Da das Gehirn von Liquor umgeben ist, wird der Bereich CSF nun durch Erosion dilatiert. Hierzu werden die Voxel der Klassen GM, WM und AT in der Nachbarschaft von CSF erodiert und CSF zugeordnet. CSF wird hierdurch indirekt dilatiert.

Durch ein anschließendes 3D Region-Growing der Gehirnregion wird in einer Ausführungsform dieser Bereich erfasst und alle übrigen (im extrazerebralen Bereich liegenden) Voxel der Klassen GM, WM und AT nach CSF umgelabelt. Der extrazerebrale Bereich ist nun komplett CSF zugeordnet. Innerhalb der Gehirn-Region werden alle Voxel der Klasse AT der Klasse WM zugeordnet, da es sich um fehlklassifizierte Fettgewebsvoxel handelt, die innerhalb des Gehirns nicht auftreten können. Da im Original-MRT-Bilddatensatz die Grauwerte des Fettgewebes denen der weißen Substanz ähnlich sind, werden AT -Voxel nach WM umgelabelt.

### Schritt 4:

Da die Gehirnregion zur Trennung des extrazerebralen Bereiches durch die Erosion verkleinert wurde, wird dies in einer Ausführungsform der Erfindung nun durch Dilatationsoperationen wieder rückgängig gemacht. Da die weiße Substanz des Gehirns (WM) durch einen dünnen Kortex der grauen Substanz (GM) umgeben ist, durch die Erosion aber dieser GM-Bereich gegebenenfalls an einigen Stellen komplett erodiert wurde, wird zunächst der Bereich der Klasse WM in den Bereich CSF hinein dilatiert. Bei dieser Dilatation werden zudem wieder Voxel, die vor der Erosion der Klasse AT angehörten der Klasse WM zugeordnet. Anschließend wird der Cortex rekonstruiert, indem der Bereich der Klasse GM in den Bereich CSF hinein dilatiert wird. Zum Schluss werden noch alle Voxel der Region AUGE ebenfalls CSF zugeordnet. Das Gehirn ist nun wieder rekonstruiert und der extrazerebrale Bereich komplett der Klasse CSF zugeordnet.

### Schritt 5:

Nun wird in einer Ausführungsform der Erfindung der Bereich CSF in der extrazerebralen Region reduziert. Hierzu wird in jeder transaxialen Schicht ausgehend von den zum Hintergrund außerhalb des Kopfes (BG) benachbarten CSF-Voxeln die Klasse SB vom Randbereich des Kopfes aus in Richtung Gehirn dilatiert. Hierzu werden CSF-Voxel, die in derselben Schicht ausschließlich von Nachbarvoxeln der Klassen BG, CSF oder SB umgeben sind, in die Klasse SB umgewandelt. Anschließend wird die Nachbarschaft in 3D betrachtet. SB-Voxel in der Nachbarschaft zu GM oder WM werden in CSF-Voxel umgewandelt, da um das Gehirn herum Liquor sein muss. Der extrazerebrale Bereich ist nun SB zugeordnet und der zerebrale Bereich den Klassen GM, WM und CSF.

### Schritt 6:

Probleme können im Bereich der Gehörgänge auftreten, da dieser als BG klassifizierte Bereich der Gehörgänge zudem mit dem wirklich außerhalb des Kopfes liegenden Bereich des Hintergrundes verbunden sein kann. Auf der anderen Seite liegen die Gehörgänge in der Nachbarschaft zu den Mastoidzellregionen und können mit diesen ebenfalls als BG klassifizierten, großräumigen Regionen verbunden sein. Dies führt dazu, dass ein großer Bereich innerhalb des Kopfes fälschlicherweise als außerhalb des Kopfes liegend (da BG zugehörig) betrachtet wird. Da der Gehörgang in der Nähe zum wirklichen Hintergrund eine eher kleine Ausdehnung hat, wird dieser Bereich in einer Ausführungsform zunächst geschlossen, indem die Klasse SB in den Bereich BG hinein dilatiert wird. Dadurch werden natürlich auch die Randbereiche des Kopfes in den Hintergrund hinein dilatiert. Durch ein anschließendes 3D Region-Growing im außerhalb des Kopfes liegenden Bereich der Klasse BG kann der Hintergrund erfasst werden. Der durch das Region-Growing nicht erfasste Bereich der BG-Voxel und dilatierten SB-Voxel wird nun nach TEMP umgelabelt. Die Vergrößerung des Kopfbereiches wird rückgängig gemacht, indem der Bereich BG in den Bereich TEMP hinein dilatiert wird. Die verbleibenden TEMP Voxel werden anschließend nach SB umgelabelt und der extrazerebrale Bereich ist vollständig segmentiert.

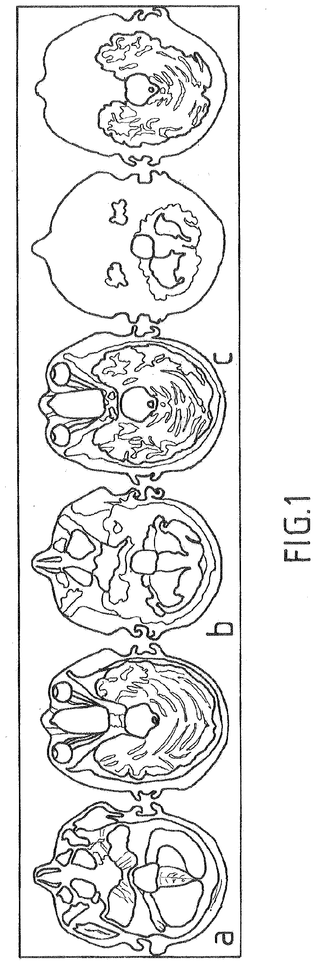

Es hat sich herausgestellt, dass besonders gute Ergebnisse erzielt werden, wenn die genannte Reihenfolge der Schritte eingehalten wird. Wird die genannte Reihenfolge nur teilweise eingehalten, so wird zwar kein optimales aber dennoch ein im Vergleich zum Stand der Technik verbessertes Ergebnis erhalten.
Es wird als Ergebnis ein Datensatz erhalten, in dem die zerebrale Region in die verschiedenen Hirngewebeklassen unterteilt ist (Klassenbild, in dem jedes Voxel des zerebralen Bereiches einer der Klassen graue Substanz (GM), weiße Substanz (WM) und Liquor (CSF) zugeordnet ist und in dem jedes Voxel des extrazerebralen Kopfbereiches der Klasse Scalp/Bone (SB) zugeordnet ist und jedes nicht zum Kopf gehörige Voxel der Klasse Hintergrund (BG) zugeordnet ist. Figur 1 verdeutlicht die Resultate. Die beiden Abbildungen a zeigen Schnitte eines T1-gewichteten Eingangsbilddatensatzes. Die beiden Abbildungen b zeigen das hieraus erhaltene Ergebnis der Gewebeklassifikation. Die beiden Abbildungen c zeigen die daraus erhaltenen Ergebnisse der zerebralen und extrazerebralen Separation.

Nachfolgend wird von einem erhaltenen ersten Datensatz 1 in Bezug auf einen menschlichen Kopf ausgegangen, der aus einer Gewebeklassifikation resultierte. Der Datensatz 1 ist ein Gewebeklassenbild, in dem jedes Voxel einer der Gewebeklassen graue Substanz (GM), weiße Substanz (WM), Liquor (CSF), Fettgewebe (AT) und Hintergrund (BG)) zugeordnet ist. Außerdem wird von einem zweiten Datensatz 2 ausgegangen, der aus einer Trennung von zerebraler und extrazerebraler Region resultierte. Bei dem Datensatz 2 handelt es sich um ein Klassenbild, in dem jedes Voxel des Gehirns einer der Klassen graue Substanz (GM), weiße Substanz (WM) und Liquor (CSF), jedes Voxel des extrazerebralen Kopfbereiches der Klasse (SB) und jedes nicht zum Kopf gehörige Voxel der Klasse Hintergrund zugeordnet ist.

Es wird anschließend der extrazerebrale Bereich des Kopfes segmentiert, so dass jedes Voxel dieses Bereiches einer der Höhlenbereiche (Klassen MASTOID, SINUS1-SINUS4), dem Knochen (Klasse BONE) oder dem extrazerebralen Weichteilgewebe (KLASSE SOFT) zugeordnet wird:
Region im Schläfenbein [lat, Os temporale]:
   Mastoidzellregion [lat, Processus mastoideus] (Klasse: MASTOID);
Höhlen im Gesichtsschädel;
   Stirnhöhle [lat, Sinus frontalis] (Klasse: SINUS1), der Region Nasenhöhle [lat, Cavum nasi], Siebbeinzellen [lat, Cellulae ethmoidales] und Keilbeinhöhle [lat, Sinus sphenoidalis] (Klasse: SINUS2),
   Kiefernhöhle [lat, Sinus maxillaris] (Klasse: SINUS3) sowie
   Rachen [lat, Pharynx] (Klasse: SINUS4);
Knochenregionen:
   Knochen des Hirnschädels [lat, Neurocranium] und des Gesichtsschädels [lat, Viscerocranium] (Klasse: BONE)
Weichteilregion:
   Weichteilgewebe des extrazerebralen Kopfbereiches, also z.B. Muskeln, Fettgewebe, Haut etc. (Klasse: SOFT)

Vorzugsweise wird wie folgt vorgegangen.

Zunächst wird die Region des Gehirns (Groß- und Kleinhirn, Hirnstamm) wie folgt bearbeitet: Auf Basis von Datensatz 2 werden alle Voxel der Klassen WM und GM zur Klasse WMGM zugeordnet, da die Schwächungseigenschaften dieser Bereiche als gleich betrachtet werden. Alle Voxel der Klasse CSF werden zunächst beibehalten, da diese für die Rekonstruktion der Schädelknochenbereiche wichtig sind. Zum Schluss können die Voxel der Klassen WMGM und CSF aber noch einer einheitlichen Klasse BRAIN zugeordnet werden, die dann einen Schwächungswert erhält.

Die extrazerebrale Region des Kopfes ist durch alle Voxel der Klasse SB im Datensatz 2 gegeben, In diesem Bereich wird die Klassenzugehörigkeit aller Voxel im Datensatz 1 betrachtet. Hohlraumregionen und Knochenregionen besitzen im Datensatz 1 im Wesentlichen die Klassenzugehörigkeit BG oder CSF, die extrazerebralen Weichteilregionen besitzen im Wesentlichen die Klassenzugehörigkeit GM, WM oder AT, die nun zu einer Klasse SOFT zusammengefasst werden. Unter Verwendung dieser Klassenzugehörigkeiten wird zur Segmentierung der extrazerebralen Region in schwächungsrelevante Bereiche wie folgt vorgegangen:

Zunächst werden die beiden Mastoidzellregionen (MASTOID) detektiert und segmentiert. Anschließend werden in der Reihenfolge Stirnhöhle (SINUS1), Nasenhöhle, Siebbeinzellen und Keilbeinhöhle (SINUS2), Kiefernhöhlen (SINUS3) sowie Rachen (SINUS4) die Höhlen im Gesichtsschädel detektiert und segmentiert. Bei der Detektion fließt dabei das anatomische Wissen über die Lagerelationen dieser Regionen zueinander ein. Unter Segmentierung ist dabei die Zuordnung jeden Voxels dieses Bereiches zu der entsprechenden Klasse zu verstehen, Bei der Segmentierung fließt die Klasseneigenschaft der Voxel in Datensatz 1 ein, entweder zur Klasse CSF oder BG zu gehören.

Anschließend wird der Bereich der Knochen (BONE) rekonstruiert. Hierbei fließt das anatomische Wissen über die Lagerelationen der Knochenregionen zur Gehirnregion (s.o. CSF umgibt das Gehirn (Groß- und Kleinhirn, Hirnstamm) im Datensatz 2) und zu den bereits detektierten Hohlräumen (Mastoidzellregion, Hohlräume im Gesichtsschädel) zur Detektion mit ein. Unter Segmentierung ist dabei die Zuordnung jeden Voxels dieses Bereiches zu der entsprechenden Klasse zu verstehen. Bei der Segmentierung fließt die Klasseneigenschaft der Voxel in Datensatz 1 ein, entweder zur Klasse CSF oder BG zu gehören sowie die Verbundenheit aller Hirn- und Gesichtsschädeiknochen.

Zum Schluss werden alle verbleibenden Voxel, die nicht einer der Klassen MASTOID, SINUS1-4, BONE oder SOFT zugeordnet sind und gleichzeitig dem extrazerebralen Bereich in Datensatz 2 angehören, der Klasse SOFT zugeordnet. Die Klassen SINUS] -4 können zum Schluss alternativ in eine Klasse SINUS zusammengefasst werden. Ebenso kann die Klasse CSF mit GMWM zu einer Klasse BRAIN verschmolzen werden.

Die einzelnen Schritte der Detektion und Segmentierung werden nun noch für die zu extrahierenden Regionen in der Reihenfolge ihrer Extraktion detaillierter beschrieben:

### Schritt 1 ;

Die im extrazerebralen Bereich (Datensatz 2) gelegenen Bereiche der Klasse BG (Datensatz 1) werden in den Bereich der Klasse CSF (Datensatz 1) hinein dilatiert, sofern die CSF-Voxel im extrazerebralen Bereich (Datensatz 2) liegen, da die BG-Regionen auf jeden Fall zu Hohlräumen oder Knochen gehören und die CSF-Bereiche um die BG-Bereiche herum aufgrund von Partialvolumeneffekten auch noch zu Hohlräumen oder Knochen gehören. CSF-Voxel, die nicht in der Nachbarschaft von BG-Bereichen liegen, gehören dagegen eher nicht zu Hohlräumen oder Knochen und werden daher nun (zunächst) der Klasse SOFT zugeordnet. Anschließend wird der Bereich BG wieder erodiert, um die miteinander verbundenen Regionen zu separieren. Der durch die Erosion entstehende Randbereich wird hierzu zunächst einer temporären Klasse TEMP zugeordnet.

### Schritt 2:

Nun werden als erste Regionen die beiden Mastoidzellregionen detektiert und segmentiert. Diese Regionen liegen im Kopf lateral/posterior des Gehörganges. Daher erfolgt die Detektion mittels eines der anatomischen Region angemessen großen und geformten (hier quadratischen) transaxialen Templates der Klasse BG, Dieses Template wird im unteren / hinteren Bereich der Matrix (anatomisch: caudal / posterior) von hinten ausgehend auf der linken und rechten Seite (anatomisch: lateral) solange verschoben, bis auf beiden Seiten jeweils unabhängig voneinander die Region der Klasse BG gefunden ist, bei der die größte Anzahl der Voxel des BG-Templates mit Voxeln der BG-Region überlagert ist. Alle vom Template überlagerten Voxel der Klasse BG (Datensatz 1) auf der rechten und linken Seite werden mit dem Klassenlabel MASTOID versehen. Der Bereich MASTOID wird anschließend durch Dilatation in den Bereich BG hinein dilatiert, um die gesamte Mastoidzellregion zu erfassen.

### Schritt 3:

Als nächste Region wird die Stirnhöhlenregion detektiert und segmentiert. Diese Region liegt im Kopf median/cranial/anterior. Daher erfolgt die Detektion mittels eines der anatomischen Region angemessen großen und geformten (hier rechteckig, schmal und lang in lateraler Richtung) transaxialen Templates der Klasse BG. Dieses Template wird im oberen / vorderen Bereich der Matrix (anatomisch: cranial / anterior) von vorne ausgehend zwischen den Template-Positionen der MASTOID-Templates (also im medianen Bereich) solange verschoben, bis die Region der Klasse BG gefunden ist, bei der die größte Anzahl der Voxel des BG-Templates mit Voxeln der BG-Region überlagert ist. Alle vom Template überlagerten Voxel der Klasse BG (Datensatz 1) werden mit dem Klassenlabel SINUS] versehen. Der Bereich SINUS] wird anschließend durch Dilatation in den Bereich BG hinein dilatiert, um die gesamte Sinus frontalis - Region zu erfassen.

### Schritt 4:

Als nächste Region wird der Bereich der Nasenhöhle, Siebbeinzellen und Keilbeinhöhle detektiert und segmentiert. Diese Region wird mit einem einzigen Klassenlabel SINUS2 versehen, da die Übergänge so groß sind, dass eine Trennung kaum möglich ist. Diese Region liegt im Kopf median/anterior und caudal (unterhalb) zur Sinus frontalis - Region. Daher erfolgt die Detektion mittels eines der anatomischen Region angemessen großen und geformten (hier rechteckig, schmal und lang in anterior-posteriorer Richtung) transaxialen Templates der Klasse BG. Dieses Template wird ausgehend von der Template-Position des SINUS1-Templates im mittleren / vorderen Bereich der Matrix von vorne / oben (anterior / cranial) ausgehend solange verschoben, bis die Region der Klasse BG gefunden ist, bei der die größte Anzahl der Voxel des BG-Templates mit Voxeln der BG-Region überlagert ist. Alle vom Template überlagerten Voxel der Klasse BG (Datensatz 1) und SINUS] werden mit dem Klassenlabel SINUS2 versehen. Der Bereich SINUS2 wird anschließend durch Dilatation (zunächst transaxial 2D, dann 3D) in den Bereich BG und SINUS] hinein dilatiert, um den gesamten Bereich der Nasenhöhle, Siebbeinzellen und Keilbeinhöhle zu erfassen.

### Schritt 5:

Nun werden als nächste Regionen die beiden Kiefernhöhlen detektiert und segmentiert. Diese Regionen liegen im Kopf median/anterior und caudal (unterhalb) zur Nasenhöhle, Siebbeinzellen und Keilbeinhöhle - Region. Daher erfolgt die Detektion mittels eines der anatomischen Region angemessen großen und geformten (hier fast quadratischen, in anterior-posteriorer Richtung etwas längeren) transaxialen Templates der Klasse BG. Dieses Template wird ausgehend von der Template-Position des SINUS2-Templates im mittleren / vorderen Bereich der Matrix von vorne / oben (anterior / cranial) ausgehend auf der linken und rechten Seite solange verschoben, bis auf beiden Seiten jeweils unabhängig voneinander die Region der Klasse BG gefunden ist, bei der die größte Anzahl der Voxel des BG-Templates mit Voxeln der BG-Region überlagert ist. Alle vom Template überlagerten Voxel der Klasse BG (Datensatz 1) und SINUS2 auf der rechten und linken Seite werden mit dem Klassenlabel SINUS3 versehen. Der Bereich SINUS3 wird anschließend durch Dilatation (zunächst transaxial 2D, dann 3D) in den Bereich BG und SINUS2 hinein dilatiert, um den gesamten Bereich der Kiefernhöhlen zu erfassen.

### Schritt 6:

Als nächste Region wird der Bereich des Rachens detektiert und segmentiert. Diese Region liegt im Kopf median/anterior und caudal (unterhalb) zur Nasenhöhle, Siebbeinzellen und Keilbeinhöhle - Region sowie hinter dem Bereich der Kiefernhöhlen. Daher erfolgt die Detektion mittels eines der anatomischen Region angemessen großen und geformten (hier quadratischen) transaxialen Templates der Klasse BG. Dieses Template wird ausgehend von der Template-Position des SINUS2-Templates, hinter (posterior) der mittleren Position der SINUS3-Templates im unteren / vorderen Bereich der Matrix von vorne / oben (anterior / cranial) ausgehend solange verschoben, bis die Region der Klasse BG gefunden ist, bei der die größte Anzahl der Voxel des BG-Templates mit Voxeln der BG-Region überlagert ist. Alle vom Template überlagerten Voxel der Klasse BG (Datensatz 1) werden mit dem Klassenlabel SINUS4 versehen. Der Bereich SINUS4 wird anschließend durch Dilatation in den Bereich BG und SINUS3 hinein dilatiert, um den gesamten Bereich des Rachens zu erfassen.

### Schritt 7:

Da mit Dilatationen gearbeitet wurde, ist noch nicht sichergestellt, dass der gesamte Bereich der im Gesichtsschädel liegenden Höhlen vollständig erfasst ist. Daher werden nun mögliche Lücken zwischen den Regionen geschlossen. Dies sind noch nicht mit einem Label versehene Bereiche der Klasse BG (Datensatz 1) zwischen den Regionen SINUS1-SINUS4, Dies erfolgt durch Dilatation der mittleren Regionen, zunächst SINUS2, dann SINUS3, in den Bereich BG hinein. Anschließend wird die Region SINUS4 nach unten (caudal) durch Dilatation in den Bereich BG (Datensatz 1) hinein erweitert. Dann wird der Bereich SINUS4 innerhalb der transaxialen Orientierung nach TEMP dilatiert, da der Rachen nicht von Gesichtsschädelknochen umgeben ist (s. weiter unten).

### Schritt 8:

Da nun alle Höhlenbereiche komplett gelabelt sind, werden die noch verbleibenden extracerebralen BG-Voxel zunächst zur Vorbereitung der Knochenrekonstruktion nach TEMP umgelabelt. TEMP-Voxel des extracerebralen Bereiches, die in den Randbereichen zu dem um den Kopf herumliegenden Hintergrund BG (Datensatz 2) liegen, werden nach SOFT umgelabelt, da die Knochen innerhalb des Kopfes liegen und von Weichteilgewebe (SOFT) umgeben sind.

### Schritt 9:

Nun erfolgt die Knochenrekonstruktion ausgehend von der Mastoidzellregion und der Stirnhöhlenregion. Die TEMP-Voxel in Nachbarschaft zu MASTOID- und SINUS1-Voxeln werden in BONE umgelabelt. Diese dienen als Seed-Voxel für ein anschließendes Region-Growing in den Bereich TEMP. Hier wird ausgenutzt, dass Hirn- und Gesichtsschädelknochen zusammenhängend sind. Alle vom Region-Growing nicht erfassten TEMP-Voxel werden nun nach SOFT umgelabelt, da sie offensichtlich nicht zum Knochenbereich gehören. Die um die Regionen SINUS2-SINUS4 herumliegenden Bereiche der Klasse BONE sollen nun von der weiteren Knochenrekonstruktion ausgeschlossen werden, da der Knochen hier nicht sehr dick ist und damit nicht erweitert werden soll. Hierzu werden die Regionen SINUS2 nach caudal und SINUS3-SINUS4 in den Bereich BONE hinein dilatiert und der dilatierte Bereich anschließend nach TEMP umgelabelt.

### Schritt 10;

Nun erfolgt die Rekonstruktion des Knochens zusätzlich ausgehend von dem um das Hirngewebe (Großhirn, Kleinhirn, Hirnstamm) herum liegenden Bereich CSF (Datensatz 1), da das Gehirn im Liquor "schwimmt" und dieser von Knochen umgeben ist. Hierzu wird die CSF-Region (Datensatz 2) in den Bereich SOFT hinein dilatiert, wenn das betrachtete extrazerebrale Voxel in Datensatz 1 der Klasse BG oder CSF angehört. Dieser extrazerebrale CSF-Bereich wird nach TEMP1 umgelabelt und dient als Ausgangspunkt für die Erweiterung des Schädelknochenbereiches. Hierzu wird der Bereich TEMP1 in den Bereich BONE hinein dilatiert.

Zusätzlich werden Fehler, die zum Beispiel durch Partialvolumeneffekte entstehen können, korrigiert. Diese Fehler führen dazu, dass es Bereiche um den Liquor des Gehirns herum gibt, die als WM, GM oder AT (Datensatz 1) klassifiziert sind. Um diese Bereiche für die Knochenrekonstruktion mit zu berücksichtigen, wird nun die CSF-Region des Gehirns (Datensatz 2) an solchen Stellen zunächst in den Bereich SOFT hinein dilatiert und die dilatierten Voxel nach TEMP1 umgelabelt. Der TEMP1-Bereich wird dann weiter in den Bereich SOFT hinein dilatiert, wenn das betrachtete extrazerebrale Voxel in Datensatz 1 der Klasse BG oder CSF angehört.

Damit ist die Rekonstruktion des Knochens abgeschlossen. Alle TEMP und TEMP1 - Voxel werden zum Schluss nach BONE umgelabelt.

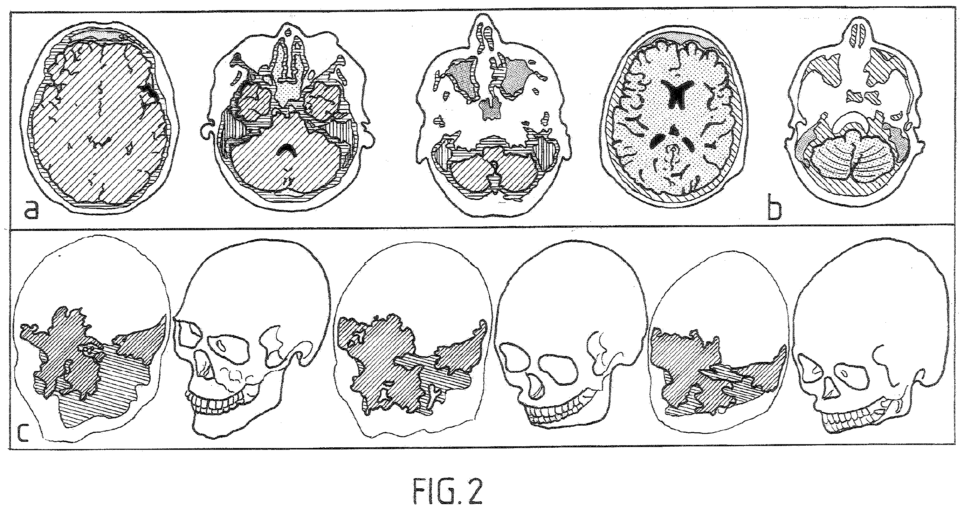

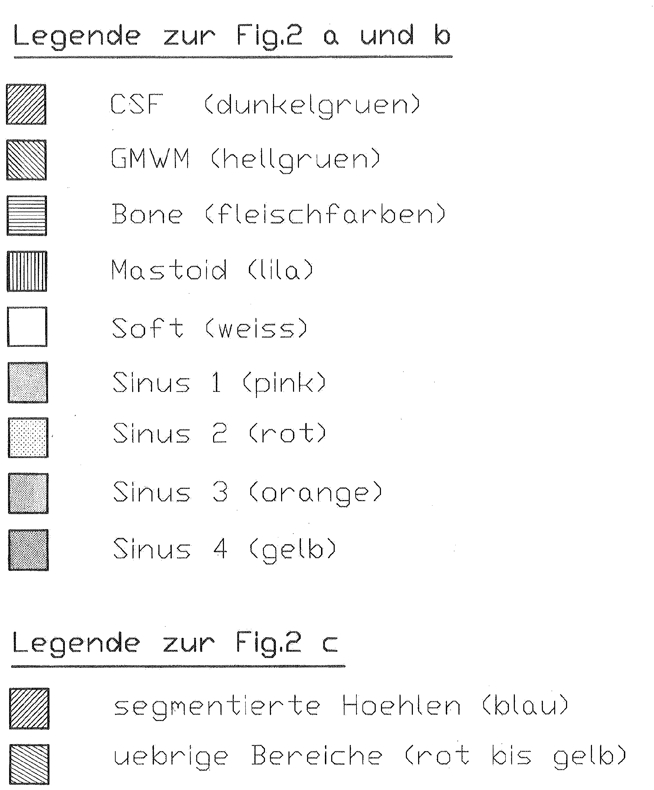

Als Ergebnis wird so ein Datensatz erhalten, in dem die unterschiedlich schwächenden Bereiche des Kopfes segmentiert sind. Bei dem Datensatz handelt es sich um ein Klassenbild bzw. Gewebeklassenbild, in dem jedes Voxel des zerebralen Bereiches einer der Klassen Hirngewebe (GMWM) und Liquor (CSF) zugeordnet ist und in dem jedes Voxel des extrazerebralen Bereiches des Kopfes einer der Klassen Mastoidzellregion (MASTOID), Hohlräume im Gesichtsschädel (SINUS1-SINUS4), Knochen (BONE) und Weichteilgewebe (SOFT) zugeordnet ist. Ein so erhaltenes Ergebnis wird in der Figur 2 gezeigt.

Die drei Abbildungen a zeigen beispielhaft Schnitte durch Klassenbilder. Hierbei sind alle zerebralen Voxel der Klasse GMWM grün dargestellt, die Voxel der Klasse CSF dunkelgrün, Im extrazerebralen Bereich sind alle Voxel der Klasse SOFT weiß, die Voxel der Klasse BONE fleischfarben dargestellt. Die Voxel der Klasse MASTOID sind lila, die Stirnhöhlenvoxel (KLASSE SINUS1) pink, die Voxel der Klasse SINUS2 rot, die Voxel der Kiefernhöhle (Klasse SINUS3) orange und die Voxel der Klasse SINUS4 sind gelb dargestellt. Die beiden Abbildungen b zeigen beispielhaft Schnitte durch Klassenbilder farbig überlagert auf die grauwertigen Originalschnitte der zugrundeliegenden T1-gewichteten Eingangsbilddaten, um die Qualität der Segmentierungsergebnisse zu verdeutlichen. Der Hintergrund (Klasse BG) ist in den Abbildungen a und b jeweils schwarz dargestellt. Die sechs Abbildungen c zeigen 3D-Darstellungen von drei segmentierten Köpfen. Zu jedem Kopf sind zwei Abbildungen zu sehen. Die jeweils linke Abbildung zeigt die Oberfläche aller segmentierten Höhlen in blau. Die übrigen Bereiche sind zur räumlichen Zuordnung der Höhlenregionen im Kopf in den Farben rot bis gelb und zudem transparent dargestellt. Die jeweils rechte Abbildung zeigt die Oberfläche der segmentierten Schädelknochen in blau. Sowohl die 2D- als auch die 3D-Abbildungen zeigen beispielhaft die sehr gute Qualität der Ergebnisse für verschiedene Eingangsbilddaten.

Alternativ kann der Bereich CSF und GMWM noch zu einer Klasse (BRAIN) zusammengefasst werden, da die Schwächungseigenschaften sich kaum unterscheiden.
Gleiches gilt für die Hohlräume im Gesichtsschädel (SINUS1-SINUS4), Diese können zu einer Region SINUS zusammengefasst werden. Da die Mastoidzellregion aus Luft- und Knochenanteilen besteht, muss diese Region gesondert betrachtet werden.

Es hat sich herausgestellt, dass besonders gute Ergebnisse erzielt werden, wenn die genannte Reihenfolge der Schritte eingehalten wird. Wird die genannte Reihenfolge nur teilweise eingehalten, so wird zwar kein optimales aber dennoch ein im Vergleich zum Stand der Technik verbessertes Ergebnis erhalten.

Das erfindungsgemäß bevorzugt eingesetzte MR/PET ist eine Vorrichtung zur Durchführung diagnostischer Untersuchungen. Eine technologische Realisierung eines MR/PET - Tomographiesystems besteht beispielsweise aus einem 3 Tesla-MRT-Gerät, in das ein sogenannter PET-Insert eingebracht ist (zum Beispiel das kommerziell erhältliche Siemens 3T MR/PET TimTrio System). Zur Steuerung des Gerätes, zur Bilddaten-Rekonstruktion und diagnostischen Auswertung der Bilddaten werden Rechnersysteme verwendet.
Um das erfindungsgemäße Verfahren durchzuführen, wurde ein Standard - PC eingesetzt.

Die Berechnungszeit auf einem PC Pentium IV, 3.4 GHz, 2GB Hauptspeicher beträgt 0,5 sec für die Stichprobenextraktion zum Training des Klassifikationsverfahrens, 12.6 sec für die Klassifikation einer 256er 3D-Matrix, 2 min 47.5 sec für die Trennung von zerebraler und extrazerebraler Region, 36.1 sec für die Segmentierung der extrazerebralen Region in Hohlräume, Knochen und Weichteilgewebe.

Bei diesem Verfahren ist keine zusätzliche CT- oder PET-Transmissionsmessung erforderlich, also keine zusätzliche Strahlenbelastung, Es sind keine nichtlinearen Registrierungen erforderlich, die bei Verwendung von Atlanten, z.B. CT-basierten Atlanten, notwendig sind.

Bei dem vorgestellten Verfahren wird die MRT-basierte Morphologiebildgebung für die PET-Schwächungskorrektur eingesetzt, indem die unterschiedlich schwächenden Bereiche aus den morphologischen Bilddaten ermittelt werden. Durch die Zuordnung geeigneter Schwächungskoeffizienten für 511 keV Strahlung zu jedem Voxel der unterschiedlich schwächenden Bereiche ("Alternative methods for attenuation correction for PET images in MR-PET scanners", E. Rota Kops, H. Herzog, IEEE NSS/MIC Conf Record 2007, pp. 4327-4330, "Magnetic resonance imaging guided attenuation and scatter corrections in three-dimensional brain positron emission tomography" , H. Zaidi, M.L. Montandon, D,O, Slosman, Med. Phys., vol, 30, pp. 937-948, 2003.) werden die segmentierten Bilddaten in Schwächungskarten konvertiert. Für die segmentierten Schwächungsbereiche des Kopfes werden aktuell folgende Werte verwendet: 0.098 1/cm für BRAIN, 0,146 1/cm für BONE, 0.096 1/cm für SOFT, 0.054 1/cm für MASTOID and 0.0 1/cm für SINUS] -4 ("Attenuation Correction in MR-PET Scanners with Segmented T1 -weighted MR images", E. Rota Kops, G. Wagenknecht et al., submitted to IEEE MIC 2009). Für die Schwächungskorrektur der PET-Bilddaten kann, wie folgt, vorgegangen werden. Die Schwächungskarten-Bilddaten werden mit den PET-Bilddaten registriert, geglättet und der Matrix- und Voxelgröße der PET-Bilddaten angepasst. Durch Vorwärtsprojektion werden sie in Schwächungskorrekturfaktoren (attenuation correction factor: ACF) umgewandelt, die bei der Rekonstuktion der PET-Emissionsbilddaten zur Schwächungskorrektur verwendet werden.

## Patentansprüche

1. Verfahren für die Ermittlung von Schwächungsbereichen in einem Organismus mit den Schritten:
- ein tierischer oder menschlicher Körper wird ganz oder teilweise mittels Magnetresonanztomographie erfasst, so dass ein MR-Bilddatensatz erhalten wird;
- die Grauwerte des MR-Bilddatensatzes werden klassifiziert derart, dass die Grauwerte Gewebeklassen zugeordnet werden,
- die auf Basis der Grauwerte klassifizierten Voxel werden mit der Anatomie des Körpers verglichen und in Abhängigkeit vom Ergebnis umklassifiziert,
- für einen Kopf wird ein erster Datensatz aus einer Gewebeklassifikation und ein zweiter Datensatz durch Trennung von zerebraler und extrazerebraler Region erhalten und basierend auf den beiden Datensätzen wird anschließend der extrazerebrale Bereich des Kopfes segmentiert, so dass jedes Voxel dieses Bereiches einem der Höhlenbereiche (Klassen MASTOID, SINUS1-SINUS4), dem Knochen (Klasse BONE) oder dem extrazerebralen Weichteilgewebe (KLASSE SOFT) zugeordnet wird,
- eine abschließend zugeordnete Klasse erhält einen der Klasse entsprechenden Schwächungswert.

2. Verfahren nach Anspruch 1, gemäß dem der Kopf mittels Magnetresonanztomographie erfasst wird, so dass ein MR-Bilddatensatz erhalten wird und die Grauwerte des MR-Bilddatensatzes als graue Substanz, weiße Substanz, Liquor, Fettgewebe und Hintergrund interpretiert und entsprechend zugeordnet werden.

3. Verfahren nach einem der vorhergehenden Ansprüche, bei dem der tierische oder menschliche Körper ganz oder teilweise mittels Positronen-Emissions-Tomographie erfasst wird und zwar vorzugsweise in einer MR/PET-Vorrichtung.

4. Verfahren nach einem der vorhergehenden Ansprüche, bei dem die Voxel ermittelt werden, die innerhalb des Körpers liegen und die als Hintergrund klassifiziert worden sind, und diese Voxel neu in Abhängigkeit von der bekannten Anatomie des Körpers klassifiziert werden.

5. Verfahren nach einem der vorhergehenden Ansprüche, bei dem zur Trennung der extrozerebrolen Region von der zerebralen Region eines Kopfes die beiden Augenregionen segmentiert werden, bevor andere Bereiche des Kopfes segmentiert werden.

6. Verfahren nach dem vorhergehenden Anspruch, bei dem im Anschluss an die Segmentierung der Augenregionen eine Hirnregion durch Erosion getrennt und durch Region-Growing erfaßt wird.

7. Verfahren nach dem vorhergehenden Anspruch, bei dem im Anschluss an die Erosion das Gehirn durch Dilatation rekonstruiert wird.

8. Verfahren nach dem vorhergehenden Anspruch, bei dem im Anschluss an die Rekonstruktion des Gehirns der das Gehirn umgebende Liquor-Bereich rekonstruiert wird und der extrazerebrale Bereich einer Klasse zugeordnet wird.

9. Verfahren nach dem vorhergehenden Anspruch, bei dem unter Berücksichtigung der Lagerelationen der anatomischen Regionen zueinander die beiden Mastoidzellregionen und anschließend die Bereiche der im Gesichtsschädel liegenden Höhlen detektiert und segmentiert werden.

10. Verfahren nach dem vorhergehenden Anspruch, bei dem die Detektion und Segmentierung mittels Template-Matching und anschließender Dilatation durchgeführt wird.

11. Verfahren nach einem der vorhergehenden Ansprüche, bei dem
- alle Voxel, die im zweiten Datensatz dem extrazerebralen Bereich und im ersten Datensatz dem Hintergrund angehören, und noch keinem der Höhlenbereiche zugeordnet sind zugeordnet sind, zur Vorbereitung der Knochenrekonstruktion umkiassifiziert werden, und
- anschließend unter Berücksichtigung der Lagerelationen der Knochen zur Gehirnregion und zu den Hohlräumen sowie unter Berücksichtigung der Verbundenheit der Knochen, Knochen detektiert und rekonstruiert werden.

12. Verfahren nach dem vorhergehenden Anspruch, bei dem die Detektion und Segmentierung der Knochen mittels Umklassifikation, Region-Growing und Dilatation durchgeführt wird.

## Claims

1. Method for the determination of attenuation areas in an organism, comprising the steps of:
- an animal or human body is captured entirely or partially by means of magnetic resonance tomography so that an MR image data set is obtained;
- the gray values of the MR image data set are classified in such a way that the gray values are assigned to tissue classes,
- the voxels classified based on the gray values are compared with the anatomy of the body and reclassified in accordance with the result,
- for a head, a first data set is obtained from a tissue classification and a second data set is obtained by separating cerebral and the extracerebral region, and the extracerebral area of the head is subsequently segmented based on the two data sets, so that each voxel of this area is assigned to one of the cavity areas (classes MASTOID, SINUS1-SINUS4), the bone (class BONE) or to the extracerebral soft tissues (class SOFT),
- a finally assigned class comprises an attenuation value corresponding to the class.

2. Method according to claim 1, according to which the head is captured by means of magnetic resonance tomography so that an MR image data set is obtained and the gray values of the MR image data set are interpreted as gray matter, white matter, liquor, adipose tissue and background and assigned accordingly.

3. Method according to one of the preceding claims, wherein the animal or human body is captured entirely or partially by means of positron emission tomography, specifically preferably in an MR/PET device.

4. Method according to one of the preceding claims, wherein the voxels are determined which lie within the body and have been classified as background, and these voxels are reclassified in accordance with the known anatomy of the body.

5. Method according to one of the preceding claims, wherein for separating the extracerebral region from the cerebral region of a head, the two eye regions are segmented before other regions of the head are segmented.

6. Method according to the preceding claim, wherein subsequent to the segmentation of the eye regions, a brain region is separated by erosion and captured by region growing.

7. Method according to the preceding claim, wherein subsequent to the erosion, the brain is reconstructed by dilation.

8. Method according to the preceding claim, wherein subsequent to the reconstruction of the brain, the liquor area that surrounds the brain is reconstructed and the extracerebral area is assigned to a class.

9. Method according to the preceding claim, wherein in consideration of the positional relationships of the anatomical regions relative to one another, the two mastoid cell regions and then the areas of the cavities located in the facial bones are detected and segmented.

10. Method according to the preceding claim, wherein the detection and segmentation is carried out by means of template matching and subsequent dilation.

11. Method according to one of the preceding claims, wherein
- all voxels that belong in the second data set to the extracerebral area and in the first data set to the background, and which have been assigned to none of the areas of the cavities as yet, are reclassified for preparing the bone reconstruction, and
- subsequently, in consideration of the positional relationships of the bones to the brain region and to the cavities, as well as in consideration of the connection of the bones, bones are detected and reconstructed.

12. Method according to the preceding claim, wherein the detection and segmentation of the bones is carried out by means of reclassification, region growing and dilation.

## Revendications

1. Procédé de détection de zones d'affaiblissement dans un organisme, comprenant les étapes de :
- saisir complètement ou partiellement un corps humain ou animal par moyen de la résonance magnétique nucléaire, de sorte qu'on obtient un jeu de données d'image MR ;
- classifier les valeurs de gris du jeu de données d'image MR, de sorte que les valeurs de gris sont attribuées à des classes de tissu,
- comparer les voxels classifiés sur la base des valeurs de gris avec l'anatomie du corps et reclassifier ceux-ci en fonction du résultat,
- obtenir un premier jeu de données pour une tête à partir d'une classification de tissu et un deuxième jeu de données à l'aide de la séparation de la région cérébrale et de la région extra-cérébrale et segmenter ensuite la zone extra-cérébrale de la tête sur la base des deux jeux de données, de sorte que chaque voxel de cette zone est attribué à l'une des zones de cavité (classes MASTOID, SINUS 1 - SINUS 4) à l'os (classe BONE) ou au tissu mou extra-cérébrale (classe SOFT),
- attribuer à une classe finalement attribuée une valeur d'affaiblissement correspondant à la classe.

2. Procédé selon la revendication 1, selon lequel la tête est saisie par moyen de la résonance magnétique nucléaire, de sorte qu'un jeu de données d'images MR est obtenu et les valeurs de gris du jeu de données d'image MR sont interprétées comme substance grise, substance blanche, liquide céphalo-rachidien, tissu adipeux et arrière-plan et sont attribuées de manière correspondante.

3. Procédé selon l'une des revendications précédentes, dans lequel le corps humain ou animal est complètement ou partiellement saisi par moyen de la tomographie par émission de positrons, de préférence dans un dispositif MR/PET.

4. Procédé selon l'une des revendications précédentes, dans lequel les voxels, qui se trouvent à l'intérieur du corps et ont été classifiés comme l'arrière-plan, sont déterminés et ces voxels sont nouvellement classifiés en fonction de l'anatomie connue du corps.

5. Procédé selon l'une des revendications précédentes, dans lequel les deux régions des yeux sont d'abord segmentées avant de segmenter d'autres régions de la tête pour séparer la région extra-cérébrale de la région cérébrale d'une tête.

6. Procédé selon la revendication précédente, dans lequel, à la suite de la segmentation des régions des yeux, une région du cerveau est séparée par érosion et saisie par region-growing.

7. Procédé selon la revendication précédente, dans lequel, à la suite de l'érosion, le cerveau est reconstruit par dilatation.

8. Procédé selon la revendication précédente, dans lequel, à la suite de la reconstruction du cerveau, la zone de liquide céphalo-rachidien, qui entoure le cerveau, est reconstruite et la zone extra-cérébrale est attribuée à une classe.

9. Procédé selon la revendication précédente, dans lequel en prenant en considération les relations de position des régions anatomiques l'une par rapport à l'autre, les deux régions de cellules mastoïde et ensuite les zones des cavités se trouvant dans le massif osseux facial sont détectées et segmentées.

10. Procédé selon la revendication précédente, dans lequel la détection et la segmentation sont exécutées par moyen de la comparaison à des modèles standard et de la dilatation suivante.

11. Procédé selon l'une des revendications précédentes, dans lequel
- tous les voxels, qui appartiennent à la zone extra-cérébrale dans le deuxième jeu de données et à l'arrière-plan dans le premier jeu de données, et qui n'ont pas encore été attribués à une des zones de cavité, sont reclassifiés pour préparer la reconstruction d'os, et
- ensuite en prenant en considération les relations de position des os par rapport à la région cérébrale et par rapport aux cavités ainsi qu'en prenant en considération le lien entre les os, des os sont détectés et reconstruits.

12. Procédé selon la revendication précédente, dans lequel la détection et la segmentation des os sont exécutées par moyen de la reclassification, du region-growing et de la dilatation.
